# EUROPEAN PATENT SPECIFICATION

(11) **EP 1 613 372 B1**
(45) Date of publication and mention of the grant of the patent: **11.03.2009**
(21) Application number: 04726449.4
(22) Date of filing: 08.04.2004
(51) Int. Cl.: A61M 1/16, A61M 1/34

(54) **MACHINE FOR PLASMA PURIFICATION COMBINED WITH PLASMA ADSORPTION-PERFUSION BY USING A TRICOMPARTMENTAL DIALYZER**
GERÄT FÜR DIE PLASMAREINIGUNG IN KOMBINATION MIT PLASMAADSORPTION-PERFUSION DURCH VERWENDUNG EINES TRIKOMPARTIMENT-DIALYSATORS
MACHINE POUR L'EPURATION DE PLASMA COMBINEE AVEC L'ADSORPTION/PERFUSION DE PLASMA AU MOYEN D'UN DIALYSEUR A TROIS COMPARTIMENTS

(30) Priority: 16.04.2003 IT PD20030076
(43) Date of publication of application: 11.01.2006
(73) Proprietor: Nalesso, Federico, 35136 Padova (IT)
(72) Inventor: Nalesso, Federico, 35136 Padova (IT)
(74) Representative: Arth, Hans-Lothar
(86) International application number: PCT/EP2004/003788
(87) International publication number: WO 2004/091694

(56) References cited:
- EP-A- 0 139 949
- DE-A- 4 102 693
- GB-A- 2 124 511
- US-A- 4 209 392

## Description

### Technical Field

The present invention relates to a machine for plasma purification combined with plasma adsorption-perfusion by using a tricompartmental dialyzer.

### Background Art

Systemic infections, also known as sepses, caused by massive and persistent invasion of the circulatory torrent by pathogenic microorganisms or by toxins produced by such microorganisms, and septic shock are among the leading causes of mortality in intensive-care situations.

The pathogenesis of these pathological conditions is known only partially.

The various elements that seem to take part in the development of the multiple-organ dysfunction in patients in intensive care include circulating peptic substances, inflammation mediators, cytokines, bacterial products, endotoxin and other molecules.

Circulating inflammation mediators that have entered the bloodstream from inflammation sites are considered responsible for remote tissue damage and are seen as decisive factors in the multiple-organ dysfunction observed in sepsis.

In recent years, the concept of performing an extracorporeal purification treatment in order to control the development, progression and damage that this patophysiological process (inflammation mediators circulating in the blood) causes in the patient, who passes from a state of multiple-organ dysfunction to a state of multiple-organ failure, has gained footing.

Many studies have demonstrated the practical possibility to remove these mediators by high-volume hemofiltration and/or by means of a process of adsorption on a specific material (resin). The data available up to now demonstrate a significant decrease in these molecules in the blood of the patient during treatment.

Some purification techniques have been tested, and continue to be tested, in patients affected by sepsis, MODS/MOFS (Multiple Organ Dysfunction Syndrome/Multiple Organ Failure Syndrome) and septic shock; the most effective include CPFA (Coupled Plasma Filtration Absorption) and HVHF (High Volume Hemofiltration).

Another extremely important clinical problem relates to patients affected by liver failure, who inexorably, as the pathology progresses, develop kidney failure (hepatorenal syndrome) and all the complications caused by retention of liver toxins. The accumulation of albumin-bound toxins has been demonstrated during liver failure; these toxins are responsible, to variable extents, for multiple-organ dysfunction (kidney, cardiovascular instability, et cetera).

The functions of albumin for transport and as a possible purification vector have been described in albumin dialysis, in which the removal of these molecules improves the clinical condition of the patient.

The best-known and most widely used known extracorporeal device for liver function support is MARS (Molecular Adsorbents Recycling System), which uses albumin that is heterologous with respect to the patient to perform purification by adsorption and by classic dialysis.

The current literature demonstrates that this approach is capable of improving patient survival.

Moreover, this type of approach is useful in intoxications caused by exogenous pathogens that are scarcely water-soluble but are plasma protein-bound.

In all of these pathologies there is certainly an involvement of cytokines, and much of the damage that affects the various organs and systems that are not primarily involved in the basic pathological process are determined by molecular factors that circulate in the blood or are dissolved in the plasma water, if water-soluble, or albumin-bound, if they are not soluble.

### Disclosure of the Invention

The aim of the present invention is to provide a blood purification device that allows to eliminate from blood all the elements that cause in patients sepsis, septic shock, multiple-organ dysfunctions, problems related to hepatorenal syndromes, et cetera.

Within this aim, an object of the present invention is to provide a blood purification device that allows to group and utilize in the same treatment all currently known physical and chemical principles for purifying the blood of the patient.

Another object of the present invention is to provide a blood purification device that can be interfaced easily even with known dialysis devices or blood purification devices.

Another object of the present invention is to provide a blood purification device that is compact.

Another object of the present invention is to provide a blood purification device that can be manufactured with known systems and technologies.

This aim and these and other objects that will become better apparent hereinafter are achieved by a blood purification device as defined in claim 1.

### Brief description of the Drawings

Further characteristics and advantages of the present invention will become better apparent from the following detailed description of a preferred but not exclusive embodiment thereof, illustrated by way of nonlimiting example in the accompanying drawings, wherein:
Figure 1 is a diagram of a device according to the invention;
Figure 2 is a schematic view of a component of a device according to the invention;
Figures 3a, 3b and 3c are three different sectional views, taken respectively along the planes IIa-IIa, IIb-IIb, IIc-IIc, of the component of Figure 2;
Figure 4 is a functional diagram of the component of Figure 2.

### Ways of caging out the Invention

With reference to the figures, a blood purification device according to the invention is generally designated by the reference numeral 10.

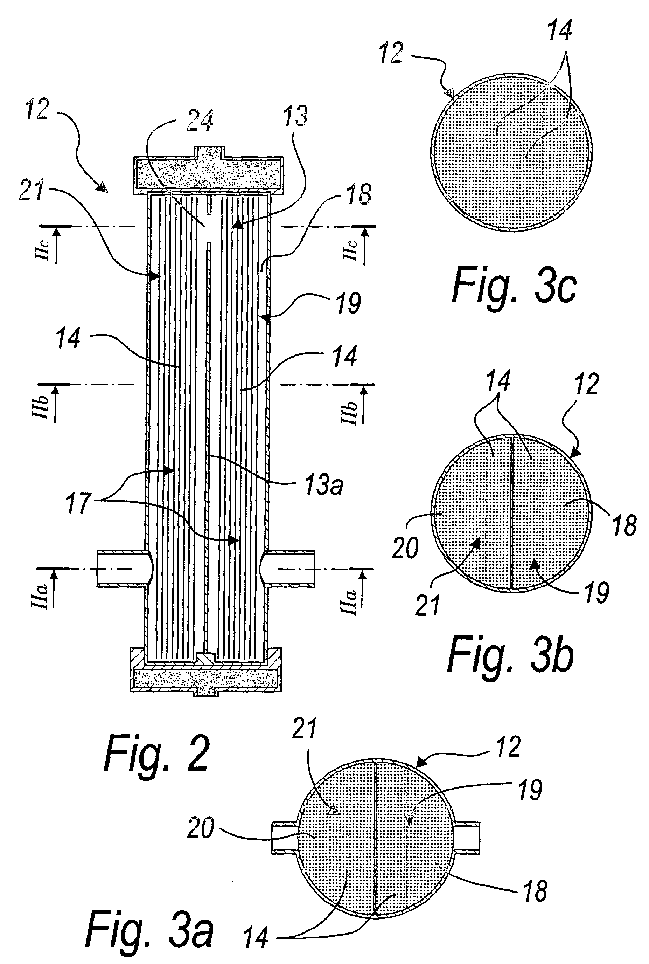

The device 10 comprises, in this described embodiment, a filter 12, of the type for hemodialysis or the like, which is constituted by an internal compartment 13 that is crossed by parallel permeable capillaries 14, of a per se known type, which are made for example of synthetic and biocompatible material such as EVAL (ethylene-vinyl-alcohol) or polypropylene (or similar materials). Reference should be made, in this regard, to Figure 2.

Whole blood flows inside the capillaries 14, which form, together with the first tube 15 in input to the filter 12 and the second tube 16 in output to the filter, a duct 17 for the flow of the whole blood.

The internal compartment 13 is divided, along the extension of the capillaries 14, into two compartments separated by a wall 13a, respectively a first compartment 18, which forms a stage 19 for filtering plasma from whole blood, and a second compartment 20, which forms a stage 21 for dialysis of whole blood by means of purified plasma, which flows in countercurrent with respect to the whole blood.

In particular, the stage 21 for dialyzing blood by means of purified plasma comprises a selectively permeable interface, which separates the whole blood stream of the duct 17 from a countercurrent stream of purified plasma that arrives from a plasma purification circuit 23, which is described in greater detail hereinafter.

In practice, such selectively permeable interface is constituted by the capillaries 14 that are present in the second compartment 20.

The first and second compartments 18 and 20 are mutually connected at the region where the countercurrent flow of the purified plasma ends.

In particular, the connection is provided by means of a hole 24 formed in the wall 13a that separates the two compartments 18 and 20.

As mentioned, the first compartment 18 forms the stage 19 for filtering plasma from the whole blood.

The stage 19 for filtering plasma from whole blood is per se of a known type.

The first compartment is functionally connected in output to the plasma purification circuit 23; such circuit in turn is functionally connected in output downstream of the stage 19 for filtering plasma from whole blood and the stage 21 for dialyzing whole blood by means of purified plasma.

The plasma purification circuit 23 comprises, for example, a device 25 for removing water-soluble and dialyzable toxic molecules of a per se known type, generally used for blood purification but used in this case in an original manner to purify plasma that arrives from the stage 19 for filtering plasma from whole blood 19.

The device 25 for removing water-soluble and dialyzable toxic molecules is composed of modules that perform diffusive processes such as high-flux dialysis (although the expression "high-flux plasma dialysis" would be more correct, since it is applied to the plasma, not to the blood), convective-diffusive processes such as hemofiltration or high-volume hemofiltration (likewise, plasma filtration and high-volume plasma filtration), processes for adsorption on a membrane (which are present, at least to a minimum extent, in all the previously cited processes).

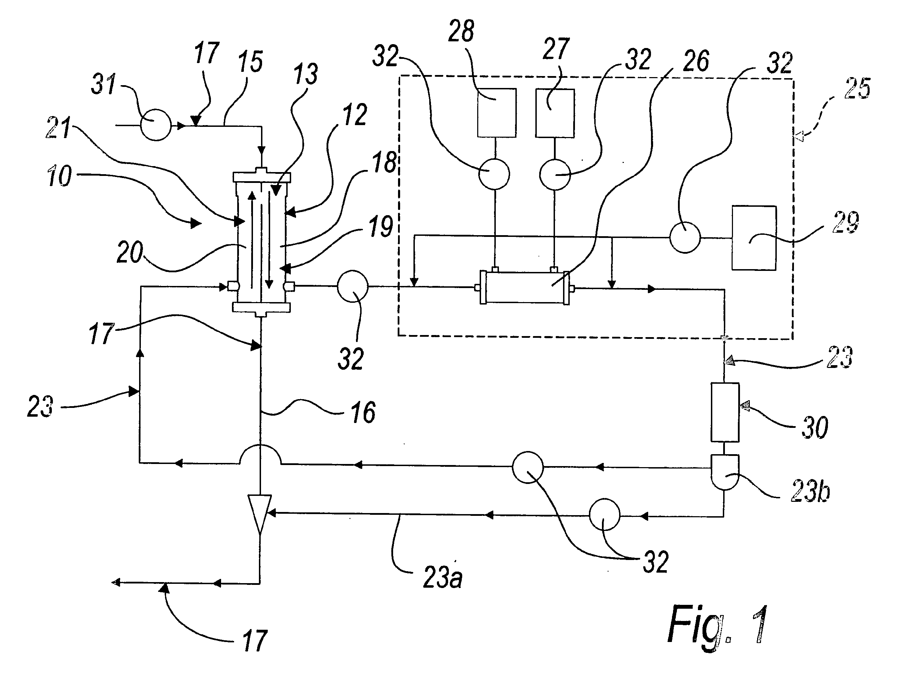

By way of example, as shown in Figure 1, the device 25 for removing water-soluble and dialyzable toxic molecules comprises a dialyzer 26 that is functionally connected to a dialysate tank 27, a tank for the used dialysate 28, and an infusate tank 29.

The plasma purification circuit 23 comprises, in series to the device 25 for removing water-soluble and dialyzable toxic molecules, a purification module of the adsorptive and/or perfusive type 30, of a per se known type, used for the purification of plasma that arrives from the device 25 for removing water-soluble and dialyzable toxic molecules.

The purification module of the adsorptive and/or perfusive type 30 comprises one or more adsorptive columns and/or one or more perfusive columns on carbon.

For circulation of the blood upstream of the filter 12 there is, for example, a hydraulic pump 31, for example of the peristaltic type.

Likewise, in the plasma purification circuit 23 there are hydraulic pumps 32, preferably of the peristaltic type, in a number and arrangement that is convenient for the particular use of the circuit.

The operation of the invention is described hereinafter.

The whole blood is drawn from the patient by means of a central venous access for hemodialysis or arteriovenous fistula and by means of the propelling force of the peristaltic pump 31 is circulated in the duct 17 for the flow of the whole blood with a flux that can vary depending on whether the device is operating in a continuous or intermittent purification mode.

The plasma, obtained by filtration from the whole blood in the stage 19 for filtering plasma from whole blood, is propelled, again by a peristaltic pump, into the plasma purification circuit 23, where it is subjected to a first purification process by performing, in the device 25 for removing water-soluble or dialyzable toxic molecules, a diffusive or convective-diffusive or pure convective method as described above.

The choice of the method performed (understood as the association of the purification processes) depends on the conditions of the patient, on the degree of purification efficiency that one wishes to obtain, and therefore on the types of molecule that one wishes to eliminate.

The first method has the goal of removing all water-soluble and dialyzable molecules by way of the membrane used in the dialyzer 26 by means of a diffusive or diffusive-convective or pure convective process and, to a small extent, by means of an adsorptive process performed on the surface of said membrane.

Once this purification step has ended, the plasma advances through the adsorptive and/or perfusive purification module 30, such as a specific column chosen according to clinical requirements, where it is subjected to an adsorption-perfusion process that allows to remove the molecules bound to plasma albumin or to other plasma components and the molecules that cannot be removed by means of the first purification method performed in the device 25 for removing water-soluble or dialyzable toxic molecules; the column can be preceded or not by a carbon column in order to increase purification. In any case, the effectiveness of molecule removal depends on the selectivity of the material used in the column.

This adsorptive process is capable of increasing the purification effectiveness of the first treatment and in any case benefits from the first treatment in performing a higher purification with respect to molecules that are scarcely or minimally removable with diffusive or convective process, even in combination.

Depending on the type of column, it is possible to perform a selective purification with respect to bilirubin, bile acids, cytokines, medium molecular weight molecules, liver toxins retained during liver failure, exogenous toxic factors and various molecules involved in the pathogenesis of sepsis and SIRS (Systemic Inflammatory Response Syndrome).

The sequential association of the two purification processes (performed in the device 25 for removing water-soluble and dialyzable toxic molecules and in the adsorptive and/or perfusive purification module 30) therefore allows a considerable increase in total purification yield.

In the clinical field, for example in case of bilirubinemia, the choice of high-flux dialysis (convective-diffusive process) allows to remove the direct bilirubin from the plasma, allowing the specific column (adsorptive-perfusive process) to remove with a higher specificity the indirect bilirubin (which is more toxic), since it has to work on a plasma that has a reduced content of direct bilirubin, which in any case reduces the adsorption of indirect bilirubin (purification synergism of the two methods used simultaneously).

Once the purification step in the adsorptive and/or perfusive purification module 30 has ended, the plasma advances along two distinct and separate paths. One fraction returns to the patient through the venous line (branch 23a of the circuit 23 that branches out from a common expander 23b, of a known type, which ensures continuity of flow), and the other fraction is directed, again under the control of a peristaltic pump, into the stage 21 for dialyzing the whole blood by means of purified plasma (second compartment 20), where by flowing in countercurrent with respect to the whole blood that is present in the capillaries 14 it acts as a dialysate and performs a selective removal of the molecules bound to plasma albumin and to the other plasma components of the whole blood contained in some of the capillaries 14 (and therefore in part of the flow duct 17), or in any case of all the molecules that can be captured by said albumin due to bond affinity.

Once the transit in the stage 21 for dialyzing the whole blood by means of purified plasma has ended, the plasma loaded with toxins (used dialysate) passes, through the hole 24, into the first compartment 18, where it joins the plasma filtered in the stage 19 for filtering plasma from whole blood, and is returned to the plasma purification circuit 23, where it undergoes the same sequential purification described earlier.

The fraction of purified plasma that returns to the patient is capable of increasing the binding capacity of the plasma with respect to all toxic factors that have binding affinity with albumin and with the other plasma components; in this manner, the plasma is capable of capturing the toxic factors that are present in the tissues and of conveying them to the dialyzer, where they are partly removed by the process of dialysis with regenerated plasma and partly removed by the purification processes performed on the plasma filtered from the whole blood.

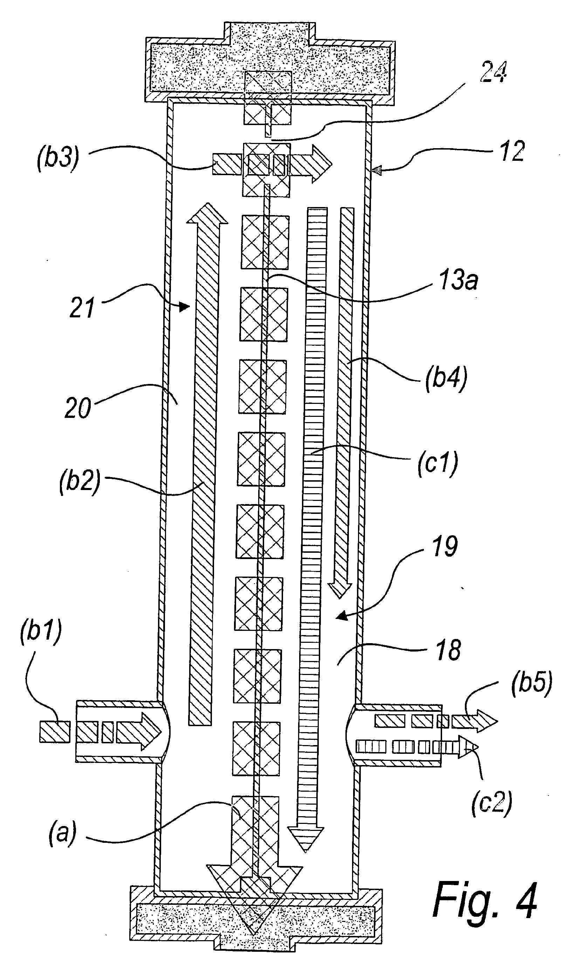

The various streams inside the filter 12 are indicated in Figure 4.

The arrow (a) designates the blood that flows in the permeable capillaries (shown in Figures 2 and 3).

The arrow (b1) designates the purified plasma that arrives from the circuit 23 that enters the second compartment 20; the arrow (b2) designates the plasma that, in countercurrent, purifies the whole blood of the capillaries (acts as a dialysate), the arrow (b3) designates the plasma which, once the capillary blood (which as such is therefore "dirty") has been purified, passes into the first compartment 18, joining the plasma filtered by the capillaries that are present in said compartment; the arrow (b4) designates the "dirty" plasma mixed with the filtered plasma; the arrow (b5) designates the "dirty" plasma in output from the filter 12 in order to enter the plasma purification circuit 23. The arrow (c1) designates the plasma filtered by the capillaries, and the arrow (c2) designates the filtered plasma, which together with the "dirty" plasma enters the plasma purification circuit 23.

The plasma of the patient therefore becomes the vector of toxic factors, which can be removed from the tissues by utilizing the binding capacity of plasma albumin and high plasma components, and the capacity of plasma water to convey water-soluble molecules.

Tissue toxic factors are captured by the plasma and entrained in the bloodstream; the invention extracts the toxic factors from the bloodstream (plasma) by means of the adsorptive-perfusive purification process, from the plasma water by means of the diffusive, convective or diffusive-convective process, and from the whole blood by means of dialysis with regenerated plasma.

The processes performed in the device 25 for removing water-soluble and dialyzable toxic molecules of the plasma purification circuit are specific for molecules that are water-soluble molecules and therefore are dissolved in the plasma water; if these processes, in addition to being diffusive, also use convection, there is a distinct increase in the removal of the molecules that have a higher molecular weight and are therefore less dialyzable by diffusion.

The processes performed in the adsorptive and/or perfusive purification module 30 allow to remove plasma albumin-bound molecules (bilirubin, skatoles, phenols, endogenous benzodiazepines, et cetera), molecules of high molecular weight which as such cannot be dialyzed (such as for example certain cytokines), and scarcely water-soluble molecules, which as such cannot be diffused by means of the plasma water.

The enormous surface of the column exposed to this process allows a high yield of the purification process.

The convective, diffusive or convective-diffusive purification processes are performed according to the standards and methods already in use and coded in international literature.

The versatility of association among the various processes performed in the device 25 for removing water-soluble and dialyzable toxic molecules and the adsorptive-perfusive process allow to obtain and perform the best treatment for the set goals (removal of cytokines in a septic patient, removal of uremic and hepatic toxins during hepatorenal syndrome, removal of bilirubin, bile acids, ammonium, skatoles, phenols, indoles, endogenous benzodiazepines during liver failure, support for patients with multiple-organ dysfunction or failure syndrome, et cetera).

The association, in the same machine, of all these purification processes allows to achieve control of acid-base and hydroelectrolytic homeostasis, maintaining a high purification of molecules involved in sepsis-SIRS (cytokines) and in multiple-organ dysfunction and failure syndrome.

The versatility, complementarity and synergism in purification of the treatments allow to support, in the same purification session, patients affected by critical pathologies that destabilize highly the entire organic homeostasis, such as hepatorenal syndrome and sepsis.

The machine can act as an intermittent treatment on demand or as a continuous therapy for support of particularly unstable patients (hepatorenal syndrome with encephalopathy, septic shock) held in intensive-care units.

The versatility of this machine also allows to provide continuous diffusive, convective and diffusive-convective treatments (CRRT: Continuous Renal Replacement Therapies) on whole blood.

To convert the machine to this operation it is in fact merely necessary to use whole blood in the plasma circuit.

The specific adsorption column selected for the treatment can be used or not on the whole blood depending on its biocompatibility and on the required purification.

In this configuration, the machine uses the plasma purification circuit for the whole blood and the device 25 for removing water-soluble and dialyzable toxic molecules is used on the whole blood.

One can conclude that the introduction of the use of the plasma of the patient (protein and plasma water portion) as a vehicle for toxic factors and as a vector of the purification process, in addition to the sequential use of the most effective and modem purification methods for purification of toxic factors, allows to develop a new purification technology that is extremely effective and versatile.

Finally, it is noted that the stage 19 for filtering plasma from whole blood and the stage 21 for dialyzing the whole blood by means of purified plasma can be provided in separate devices that are not grouped in a single hemodialysis filter (so as to form in practice a single tricompartmental dialyzer) and are divided into two compartments by a wall, as briefly described hereinafter (no figures are attached because the concept is extremely intuitive and equivalent to the structure of the purification device described above).

In practice, the stage for filtering plasma from whole blood can comprise a filter, such as a filter for plasmapheresis or the like, while the stage for dialyzing the whole blood by means of purified plasma is constituted by a compartment that is independent and completely separate from the plasmapheresis filter and is crossed by permeable parallel capillaries that delimit part of the duct where the whole blood flows.

The purified plasma flows in countercurrent within the compartment.

The compartment is functionally connected, by means of a tube, to the filter in the region where the countercurrent flow of the purified plasma ends; the region and the compartment are functionally connected by means of tubes respectively to an inlet and an outlet of the plasma purification circuit.

The invention thus conceived is susceptible of numerous modifications and variations, all of which are within the scope of the inventive concept; all the details may further be replaced with other technically equivalent elements.

In practice, the materials employed, so long as they are compatible with the specific use, as well as the dimensions, may be any according to requirements and to the state of the art.

## Claims

1. A blood purification device, comprising a duct (17) for the flow of whole blood along which there is a stage (19) for filtering plasma from the whole blood, which is functionally arrangeable in connection to a plasma purification circuit (23), and a stage (21) for whole blood dialysis by means of plasma purified in said circuit (23), said stage (21) for whole blood dialysis comprising a selectively permeable interface for separating at least part of the whole blood stream of said duct (17) from a countercurrent stream of plasma purified in said circuit (23), the device further comprising a filter (12), which is constituted by an internal compartment (13) crossed by parallel permeable capillaries (14) constituting the selectively permeable interface, the space inside said capillaries (14) delimiting at least part of said duct (17) for the flow of said whole blood, **characterized in that** said internal compartment (13) is divided, in the direction of the extension of said capillaries (14), into two separate compartments (18, 20), respectively a first compartment (18) that forms said stage (19) for filtering plasma from whole blood and a second compartment (20) that forms said stage (21) for dialyzing the whole blood by means of purified plasma in countercurrent with respect to the whole blood, said first and second compartments (18, 20) being mutually connected at the region where the countercurrent flow of said purified plasma ends, said first and second compartments (18, 20) being further functionally arrangeable in connection respectively to an input and an output of said plasma purification circuit (23).

2. The blood purification device according to claim 1, **characterized in that** it comprises a plasma purification circuit (23) filtered by said stage (19) for filtering plasma from whole blood, which is functionally connected to said stage (21) for dialyzing the whole blood by means of purified plasma, said plasma purification circuit (23) being functionally connected to said duct (17) downstream of both said stage (19) for filtering plasma from whole blood and said stage (21) for dialyzing whole blood by means of purified plasma.

3. The blood purification device according to claim 2, **characterized in that** said plasma purification circuit (23) comprises a device (25) for removing water-soluble and dialyzable toxic molecules, which is generally used to purify blood but is used to purify plasma that arrives from said stage (19) for filtering plasma from whole blood.

4. The blood purification device according to claim 3, **characterized in that** said device (25) for removing water-soluble and dialyzable toxic molecules is composed of modules for performing diffusive processes such as high-flux dialysis, convective-diffusive processes, purely convective processes, membrane-based adsorptive processes.

5. The blood purification device according to claim 4, **characterized in that** said device (25) for removing water-soluble and dialyzable toxic molecules comprises a dialyzer (26) that is functionally connected to a dialysate tank (27), a used dialysate tank (28), and an infusate tank (29).

6. The blood purification device according to claim 2 or to subsequent claims, **characterized in that** said plasma purification circuit (23) comprises an adsorptive and/or perfusive purification module (30), used to purify plasma that arrives from said device (25) for removing water-soluble or dialyzable toxic molecules.

7. The blood purification device according to claim 6, **characterized in that** said adsorptive and/or perfusive purification module (30) comprises one or more adsorption columns and/or one or more perfusion columns on carbon.

## Patentansprüche

1. Ein Gerät zur Plasmareinigung, enthaltend einen Leitungskanal (17) für den Fluss des Gesamtblutes, entlang dessen sich eine Apparatur (19) zur Plasmafiltration aus dem Gesamtblut befindet, die funktionell in Verbindung zu einem Plasmareinigungskreislauf (23) geregelt werden kann, und entlang dessen sich eine Apparatur (21) zur Dialyse des Gesamtblutes durch Plasma, das in besagtem Plasmareinigungskreislauf (23) gereinigt wurde, befindet, die besagte Apparatur (21) zur Gesamtblutdialyse enthaltend eine selektiv permeable Grenzfläche zur Abtrennung von zumindest einem Teil des Gesamtblutstroms durch besagten Leitungskanal (17) von einem Gegenströmungsfluss des in besagtem Plasmareinigungskreislauf (23) gereinigten Plasmas, das Gerät weiterhin einen Filter (12) enthaltend, der aus einer inneren Kammer (13) besteht, die von parallelen permeablen Kapillaren (14) gekreuzt wird, um die selektiv permeable Grenzfläche zu bilden, und wobei der Raum innerhalb dieser Kapillaren (14) zumindest einen Teil des besagten Leitungskanals (17) beim Fluss besagten Gesamtbluts begrenzt, **dadurch gekennzeichnet, dass** diese innere Kammer (13) entlang der Ausrichtung besagter Kapillaren (14) in zwei getrennte Kammern (18,20) unterteilt ist, wovon die erste Kammer (18) die besagte Apparatur (19) zur Plasmafiltration aus dem Gesamtblut bildet und die zweite Kammer (20) die besagte Apparatur (21) zur Dialyse des Gesamtbluts durch gereinigtes Plasma im Gegenstrom zum Gesamtblut bildet, wobei besagte erste und zweite Kammer (18, 20) in dem Bereich miteinander verbunden sind, an dem der Gegenströmungsfluss besagten gereinigten Plasmas endet, und wobei besagte erste und zweite Kammer (18, 20) zudem funktionell regelbar miteinander verbunden sind hinsichtlich des jeweiligen Inputs oder Outputs besagten Plasmareinigungskreislaufs (23).

2. Das Gerät zur Plasmareinigung gemäß Anspruch 1, **dadurch gekennzeichnet, dass** es einen Plasmareinigungskreislauf (23), der durch besagte Apparatur (19) zur Plasmafiltration aus dem Gesamtblut filtriert wurde, umfasst, der mit besagter Apparatur (21) zur Dialyse des Gesamtbluts durch gereinigtes Plasma funktionell verbunden ist, wobei besagter Plasmareinigungskreislauf (23) strömungsabwärts sowohl besagter Apparatur (19) zur Plasmafiltration aus dem Gesamtblut als auch besagter Apparatur (21) zur Dialyse des Gesamtbluts durch gereinigtes Plasma funktionell mit besagtem Leitungskanal (17) verbunden ist.

3. Das Gerät zur Plasmareinigung gemäß Anspruch 2, **dadurch gekennzeichnet, dass** besagter Plasmareinigungskreislauf (23) eine Apparatur (25) zur Entfernung wasserlöslicher und dialysierbarer toxischer Moleküle umfasst, die allgemein zur Blutreinigung verwendet wird, aber auch zur Reinigung von Plasma verwendet wird, das von besagter Apparatur (19) zur Plasmafiltration aus dem Gesamtblut stammt.

4. Das Gerät zur Plasmareinigung gemäß Anspruch 3, **dadurch gekennzeichnet, dass** besagte Apparatur (25) zur Entfernung wasserlöslicher und dialysierbarer toxischer Moleküle aus Modulen besteht, die Diffusionsprozesse wie zum Beispiel High-Flux-Dialyse, konvektiv-diffusive Prozesse, rein konvektive Prozesse und membranbasierte adsorptive Prozesse durchführen.

5. Das Gerät zur Plasmareinigung gemäß Anspruch 4, **dadurch gekennzeichnet, dass** besagte Apparatur (25) zur Entfernung wasserlöslicher und dialysierbarer toxischer Moleküle einen Dialysator (26) umfasst, der mit einem Dialysatbehälter (27), einem Behälter (28) für gebrauchtes Dialysat und einem Infusatbehälter (29) funktionell verbunden ist.

6. Das Gerät zur Plasmareinigung gemäß Anspruch 2 oder nachfolgender Ansprüche, **dadurch gekennzeichnet, dass** besagter Plasmareinigungskreislauf (23) ein adsorptives und/oder perfusives Reinigungsmodul (30) umfasst, das zur Reinigung von Plasma verwendet wird, das aus besagter Apparatur (25) zur Entfernung wasserlöslicher und dialysierbarer toxischer Moleküle stammt.

7. Das Gerät zur Plasmareinigung gemäß Anspruch 6, **dadurch gekennzeichnet, dass** besagtes adsorptive und/oder perfusive Reinigungsmodul (30) eine oder mehrere Adsorptionssäulen und/oder eine oder mehrere Perfusionssäulen an Aktivkohle umfasst.

## Revendications

1. Une machine pour l'épuration de plasma, comprenant une gaine (17) pour le flux du sang entier, le long de laquelle se trouve un appareillage pour la filtration de plasma du sang entier (19), qui peut être réglé fonctionnellement en connexion avec un circuit d'épuration de plasma (23), et le long de laquelle se trouve un appareillage pour la dialyse du sang entier (21) par le plasma épuré dans ledit circuit d'épuration de plasma (23), ledit appareillage pour la dialyse du sang entier (21) comprenant un surface de séparation sélectivement perméable pour la séparation au moins d'un part du sang entier à travers de ladite gaine (17) d'un flux contre-courant du plasma épuré dans le circuit d'épuration de plasma (23), la machine comprenant en outre un filtre (12) qui consiste d'une chambre interne (13) croisée de tubes capillaires (14) perméables parallèles pour former le surface de séparation sélectivement perméable, dont l'espace dans lesdits tubes capillaires (14) délimite au moins une partie de ladite gaine (17) au flux du ledit sang entier, **caractérisée en ce que** cette chambre interne (13) est compartimentée dans deux chambres (18, 20) séparées le long de l'extension des lesdits tubes capillaires, dont la première chambre (18) forme ledit appareillage pour la filtration de plasma du sang entier (19) et la deuxième chambre forme ledit appareillage pour la dialyse du sang entier (21) par le plasma épuré en contre-courant au sang entier, dont ladite première et ladite deuxième chambre (18, 20) sont connectées dans cette zone où le flux contre-courant de ledit plasma épuré termine, et dont ladite première et ladite deuxième chambre (18, 20) sont en outre fonctionnellement connectées, réglables concernant les flux entrants et sortants, respectivement, de ledit circuit d'épuration de plasma (23).

2. La machine pour l'épuration de plasma, selon la revendication 1, **caractérisée en ce qu'**elle comprend un circuit d'épuration de plasma (23) filtré par ledit appareillage pour la filtration de plasma du sang entier (19) qui est fonctionnellement connecté avec ledit appareillage pour la dialyse du sang entier (21) par le plasma épuré, dont ledit circuit d'épuration de plasma (23) est fonctionnellement connecté avec ladite gaine (17) en aval de ledit appareillage pour la filtration de plasma du sang entier (19) comme de ledit appareillage pour la dialyse du sang entier (21) par le plasma épuré.

3. La machine pour l'épuration de plasma, selon la revendication 2, **caractérisée en ce que** ledit circuit d'épuration de plasma (23) comprend un appareillage (25) pour éliminer des molécules toxiques hydrosolubles et dialysables qui est usé en général pour l'épuration du sang, mais peut être usé aussi pour l'épuration du plasma qui provient de ledit appareillage pour la filtration de plasma du sang entier (19).

4. La machine pour l'épuration de plasma, selon la revendication 3, **caractérisée en ce que** ledit appareillage (25) pour éliminer des molécules toxiques hydrosolubles et dialysables consiste de modules qui conduisent des processus diffusifs comme pour exemple la dialyse «high-flux», des processus convectifs-diffusifs, des processus purement convectifs et des processus adsorptifs à base de membranes.

5. La machine pour l'épuration de plasma, selon la revendication 4, **caractérisée en ce que** ledit appareillage (25) pour éliminer des molécules toxiques hydrosolubles et dialysables comprend un dialyseur (26) qui est fonctionnellement connecté avec un réservoir pour le dialysat (27), un réservoir pour le dialysat usé (28) et un réservoir pour l'infusat (29).

6. La machine pour l'épuration de plasma, selon les revendications 2 et suivantes, **caractérisée en ce que** ledit circuit d'épuration de plasma (23) comprend un module d'épuration (30) adsorptif et/ou perfusif qui est usé pour l'épuration du plasma provenant de ledit appareillage (25) pour éliminer des molécules toxiques hydrosolubles et dialysables.

7. La machine pour l'épuration de plasma, selon la revendication 6, **caractérisée en ce que** ledit module d'épuration (30) adsorptif et/ou perfusif comprend une ou plusieurs colonnes d'adsorption ou colonnes de perfusion sur charbon activé.
